# EUROPÄISCHE PATENTSCHRIFT

(11) **EP 0 608 775 B1**
(45) Veröffentlichungstag und Bekanntmachung des Hinweises auf die Patenterteilung: **21.03.2001**
(21) Anmeldenummer: 94100835.1
(22) Anmeldetag: 21.01.1994
(51) Int. Cl.: A61M 25/01

(54) **Einstückiges Führungsteil und Verfahren zum Herstellen desselben**
One piece guidewire and process for the manufacture thereof
Fil de guidage en une pièce et procédé de fabrication

(30) Priorität: 28.01.1993 DE 4302326; 15.05.1993 DE 4316330
(43) Veröffentlichungstag der Anmeldung: 03.08.1994
(73) Patentinhaber: ANGIOMED GmbH & Co. Medizintechnik KG, 76227 Karlsruhe (DE)
(72) Erfinder: Lindenberg, Josef, D-76227 Karlsruhe (DE); Schnepp-Pesch, Wolfram, D-76185 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 401 158
- EP-A- 0 515 201
- WO-A-91/15152
- FR-A- 556 912
- US-A- 4 003 369
- US-A- 4 991 602

## Beschreibung

Die Erfindung betrifft Führungsteile zur Katheterisierung, nach den Oberbegriffen des Anspruchs 1 oder 2.

Derartige Führungsteile werden in der Regel als Führungsdrähte im medizinischen Bereich zur Einführung von Teilen in den menschlichen Körper, insbesondere in Gefäßen oder Organe desselben, eingesetzt. Mit derartigen Führungsdrähten können Katheter, Schleusen, implantierbare Katheder (Stents), Kanülen oder dergleichen in den menschlichen Körper eingeführt und gegebenenfalls eingesetzt werden.

Bekannte Führungsdrähte bestehen aus einem oder mehreren wendelförmig gewickelten Einzeldrähten, wobei bei einem gewendelten Einzeldraht die Wendel in der Regel eine sehr geringe Steigung aufweist, während mehrere Drähte of mit hoher Steigung verschlugen sind. Am vorderen Einführende eines solchen Führungsdrahtes ist dieser abgestumpft und dort mit einer Perle, einer Kappe oder dergleichen versehen. Diese wird durch einen sich innerhalb der Wendel erstreckenden feinen Sicherungsdraht gehalten, mit dem sie verschweißt ist. Ein derartiger Führungsdraht ist mehrteilig, weist die die genannte sowie weitere Schweißstellen auf. Derartige Schweißstellen sind praktisch Sollbruchstellen, da sie schon bei einer geringen Belastung aufgrund von Ermüdungserscheinungen der Schweißstellen brechen können. Eine weitere Schwachstelle ist der dünne Sicherheitsfaden, da dieser nur einen sehr geringen Durchmesser aufweist. Eine andere große Gefahr besteht darin, daß an den Wendeln im Bereich zwischen zwei Windungen aufgrund des dort gegebenen spitzen Winkel des Öffnungsbereichs zwischen den beiden Windungen sich andere bei dem Einführen verwendete Teile, wie Kanülen, insbesondere wenn sie mit einem schräg zugeschliffenen Ende versehen sind, verhaken können und damit zu einem Reißen des Führungsdrahtes bzw. des oder der Wendeldrähte führen können. Es bestht eine bisher nicht auszuschließende Gefahr, daß Teile des Führungsdrahtes sich lösen und im Körper verbleiben. Derartige Fällte sind auch bekanntgeworden.

Die US-A-4,991,602 zeigt ein gattungsgemäßes Führungsteil mit einem vorderen, im Längsschnitt ovalen Einführende, einem sich daran anschließenden, sich konisch erweiternden Übergangsbereich und einem zylindrischen Hauptabschnitt. Das bekannte Führungsteil ist symmetrisch ausgebildet; sein rückwärtiges Ende weist die gleiche Kontur wie das vordere Ende auf.

Die Übergangsstelle zwischen Einführende und Übergangsbereich, also die Stelle mit geringstem Durchmesser ist relativ scharf ausgebildet. Hier stoßen teilkugelförmige, rückwärtige Abrundungen des Führungsendes, deren Kontur in diesem Bereich nahezu radial verläuft un der einen geringen Winkel zur Längsachse verlaufende - zum Einführende hin sich verjüngende - Übergangsbereich in einer scharfen Kurve zusammen.

Dies ist nachteilig, da hierdruch ein Verhaken des Führungsteil beim Zurückziehen aus einem Blutgefäß, wie durch eine Hohlnadel, einen Katheder oder dergleichen am vorderen Ende des Hohlteils erfolgen kann, wodurch die Gefahr eines Abreißens des Einführendes besteht. Eine solche Gefahr wird grundsätzlich durch den scharfen Übergang zwischen Einführende und konischem Übergangsbereich erhöht, da die verjüngte Stelle aufgrund des scharfen Übergangs geschwächt ist. Eine weitere Schwächung tritt dadurch auf, daß Grund dieser Ausgestaltung bei Abbiegungen eines solchen Grades beim einführen, hier einer Materialermüdung, auftreten kann; die Übergangsstelle zwischen Einführende und konischem Übergangsbereich ist bei dem bekannten Gegenstand praktisch als Solldruckstelle ausgestaltet.

Der Erfindung liegt daher die Aufgabe zugrunde, den bekannten Stand der Technik dahigehend zu verbessern, daß bei progressiv zum vorderen Ende hin ansteigende Flexibilität des Führungsgrades die Gefahr von Beschädigung desselben sowie Verletzungen der Person, in die der Führungsgrad eingeführt wird, ausgeschlossen wird.

Zur Lösung der genannten Aufgabe sieht die Erfindung bei gattungsgemäßen Führungsteilen vor, daß das Einführende die Form eines Tropfens aufweist, der an seinem rückwärtigen Ende stetig in den Übergangsbereich übergeht.

Ein Tropfen weist ausschließlich eine Außenkontur mit kontinuierlichen Krümmungen, also über seine gesamte Form hin einen endlichen Krümmungsradius auf. Darüber hinaus läuft ein Tropfen in einer wesentlich geringeren Krümmung, also relativ großen keine Kanten bildenden Krümmungsradius in das vordere Ende des zur erweiterten Spitze hin verjüngenden Übergangsbereichs ein, so daß dieser Bereich beim Erfindungsgegenstand weicher, stetiger, als beim Stand der Technik ist.

Der erfindungsgemäße Führungsraht ist vollständig einstückig, d.h. aus einem Stück hergestellt. Damit werden "Sollbruchstellen", wie sie bei bekannten Führungsdrähten, insbesondere durch Schweißstellen, gegeben sind, vermieden und damit die hierdurch gegebene Gefahr eines Bruchs oder Abreißens von Teilen des Führungsdrahtes an solchen Stellen vollständig ausgeschlossen. Der einstückige Führungsdraht kann darüber hinaus im Gegensatz zu gewendelten Führungsdrähten mit einer glatten Oberfläche ohne Hinterschneidungen oder Außenkonturbereiche mit spitzen Winkeln ausgebildet sein, wie sie insbesondere bei gewendelten Führungsdrähten zwischen zwei benachbarten Windungen gegeben sind. Soweit der erfindungsgemäße Führungsdraht Verjüngungen oder Erweiterungen aufweist, so verlaufen diese stetig und kontinuierlich. Um dem Führungsdraht insbesondere in seinem vorderen Einführbereich die gewünschte sehr hohe Flexibilität, d.h. die große, aber reversible und damit elastische Weichheit und Biegefähigkeit zu verleihen, sind in diesem Bereich verjüngte Abschnitte vorgesehen, d.h. Abschnitte mit gegenüber den Maximalquerabmessungen des sonstigen Führungsdrahtes reduzierten Querabmessunge. Aufgrund des elastischen Metallmaterials, das Edelstahl sein kann, vorzugsweise aber eine hochzugfeste tita-Nickel-Legie-(Nitinol) ist, können die Querschnittsabmessungen in den Bereichen minimalen Durchmessers sehr gering gewählt werden, ohne daß die Zugfestigkeit unzulässig reduziert wird. Aber auch andere Metallegierungen, wie Biometall oder dergleichen, kommen in Frage. Gemäß bevorzugter Ausgestaltungen ist vorgesehen, daß der Minimaldurchmesser weniger als ein Drittel des Maximaldurchmessers beträgt und insbesondere daß der Minimaldurchmesser weniger als ein Viertel des Maximaldurchmessers beträgt.

Durch die glatte Wandung und das harte Metallmaterial wird ausgeschlossen, daß die Spitze einer scharfen Kanüle sich verhakt oder in das Material eindringt und blockiet und/oder beschädigt wird.

Die Querabmessunge können derart ausgestaltet sein, daß der vom Einführende zum rückwärtigen Bereich verlaufende Übergangsbereich sich kontinuierlich erweitert, wobei er insbesondere konisch ist. Hinsichtlich der Länge des Übergangsbereiches sieht eine bevorzugte Ausgestaltung vor, daß der sich von der Stelle geringsten Querschnitts hinter dem Einführende von diesem forterstreckende Übergangsbereich mindestens zehnmal so lang ist wie der Abschnitt von der Stelle geringsten Querschnitts unmittelbar hinter dem Einführende bis zur Stirnseite desselben. In alternativer Ausgestaltung kann zur Erhöhung der Biegefähigkeit des Führungsdrahtes in gewünschten Bereichen vorgesehen sein, daß der Übergangsbereich periodisch sich erweiternde und verjüngende Abschnitte aufweist. Hier kann die Länge der Periode von sich erweiternden und verjüngenden Abschnitten in geeigneter Weise gewählt werden, wobei die Periode der sich erweiternden und verjüngenden Abschnitte eine Länge zwischen 3 und 9 mm hat.

Eine bevorzugte Ausgestaltung sieht vor, daß der Führungsdraht in seinem vorderen, von seinem Einführende aus verlaufenden Bereich abgeätzte Abschnitte aufweist. Grundsätzlich liegt ein wesentlicher und eigener Erfindungsgedanke darin, daß ein einstückiger erfindungsgemäßer Führungsdraht über seine Länge hin abgetragene, insbesondere abgeätzte Bereiche aufweist, in denen er also gegenüber seinen normalen und ursprünglichen Querabmessunge in seiner Querabmessung durch Ätzen reduziert ist.

Der Führungsdraht kann in bevorzugter Weise derart ausgebildet sein, daß er zumindestens in seinem vordern Bereich in Abhängigkeit von der Temperatur oberhalb bzw. unterhalb eines vorgegebenen Temperaturbereichs eine unterschiedliche Konfiguration einnimmt. Ein solcher Führungsdraht weist also Memory-Eigenschaften auf. Er kann beispielsweise aus Memory-Metall bestehen, d.h. aus einem Metall, wie eben insbesondere Nickel-Titan-Legierungen, die bei Änderungen der Temperaturen einen Rückstelleffekt in anderer Konfiguratin oder Kontur aufweisen. So kann ein derartiger Führungsdraht in seinem Tieftemperaturzustand, d.h. unterhalb der Übergangstemperatur, gerade ausgebildet sein und bei Erhöhung der Umgebungstemperatur oder seiner eigenen Temperatur über die Übergangstemperatur hin eine bogenförmige Abbiegung ausbilden und vice versa, je nachdem, welche Kontur unter welchen Bedingungen gewünscht ist.

Das vordere Einführende des Führungsdrahtes kann in verschiedener Weise ausgebildet sein. Zunächst wird vorteilhafterweise grundsätzlich vorgesehen sein, daß das Einführende in seinem nach vorne gerichteten, freien Bereich zumindestens teilkugelförmig ausgebildet ist.

Verfahren zur Herstellung erfindungsgemäße einstückiger Führungsdrähte sehen vor, daß ein stabförmiges, einstückiges Teil mit glatter Außenwandung aus flexiblem Metall in Teilbereichen zur Erzeugung von Querschnittsverminderungen abgeschliffen wird oder daß die Querschnittsverminderungen mit kontinuierlichen Übergangsbereichen hergestellt werden kann dadurch weitergebildet sein, daß vor dem Schritt des Ätzens zumindestens Teilbereiche des stabförmigen Teils mit einer gegen das Ätzbad resistenten Beschichtung versehen werden. In weiterer Ausbildung kann vorgesehen sein, daß das stangenförmige Teil mit konstanter Geschwindigkeit aus dem Ätzbad heruasgezogen wird und daß das stabförmige Teil zumindestens durch eine das Ätzbad gegen eine nichtätzende Umgebund abdichtende Dichtung hindurchgezogen wird, wobei entweder ein massives stabförmiges Teil aus dem Ätzbad herausgezogen wird oder ein rohrförmiges Teil aus dem Ätzbad herausgezogen wird.

Eine weitere bevorzugte Ausgestaltung sieht vor, daß die Temperatur des Ätzbades konstant gehalten wird.

Mit der Erfindung wird ein Führungsdraht hoher Flexibilität geschaffen, bei dem die Härte, insbesondere die Zugfähigkeit des Materials, in geeigneter Weise ebenso eingestellt werden kann wie die Biegefähigkeit des vorderen Einführbereiches des Führungsdrahtes durch geeignete Wahl der Verjüngungen und Verdickungen des Drahtes. Darüber hinaus weist der Führungsdraht aufgrund seiner einstückigen Ausgestaltung aus einem stab- oder rohrförmigen Teil eine wesentlich höhere Torsionsfähigkeit auf, als dies bei bekannten gewickelten Führungsdrähten der Fall ist. Eine Rotationsbewegung am rückwärtigen Ende des Führungsdrahtes wird praktisch unmittelbar und vollständig durch das Einführende ebenfalls ausgeführt.

Weitere Vorteile und Merkmale der Erfindung ergeben sich aus den Ansrüchen und aus der nachfolgenden Beschreibung, in der zwei bevorzugte Ausführungsbeispiele des erfindungsgemäßen Führungsdrahtes unter Bezugnahme auf die Zeichnung im einzelnen erläutert sind. Dabei zeigt:
- Figur 1: eine erste bevorzugte Ausgestaltung eines erfindungsgemäßen Führungsdrahtes; und
- Figur 2: eine weitere Ausgestaltung eines erfindungsgemäßen Führungsdrahtes.

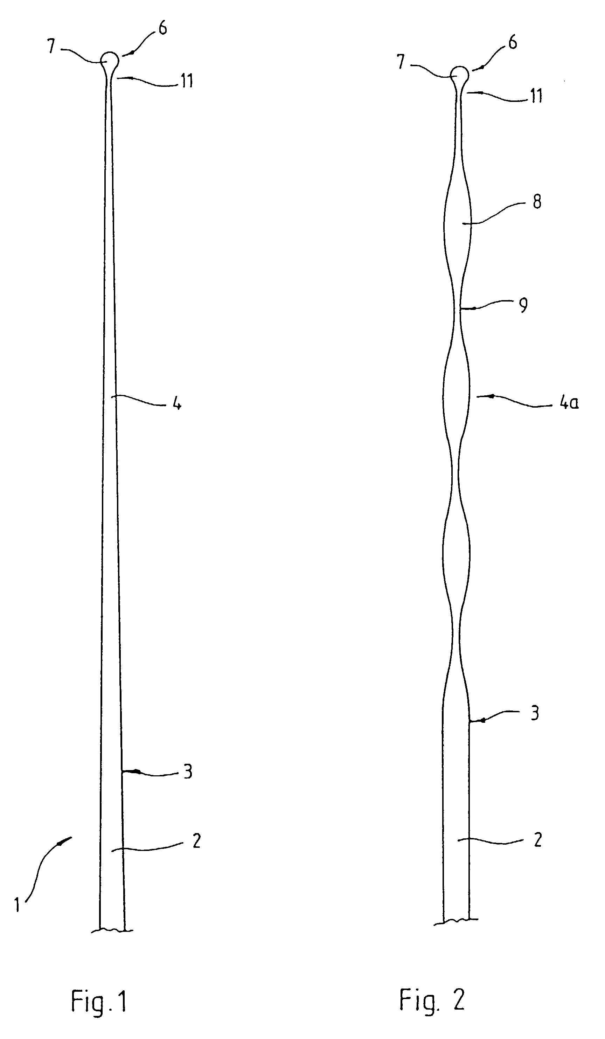

Das erfindungsgemäße Führungsteil in Form eines Führungsdrahtes 1 der Figur 1 weist einen rückwärtigen Hauptbereich 2 auf, der sich von seinem freien Ende (nicht dargestellt) bis zu den Pfeilen 3 erstreckt und über seine gesamte Länge, die mehrere Dezimeter bis über 1 m betragen kann, einen konstanten Querschnitt aufweist, der im dargestellten Ausführungsbeispiel kreisförmig ist. Von den Pfeilen 3 aus verjüngt sich der Führungsdraht in einen Übergangsbereich 4 bis nahe zu seinem vorderen Einführungsende 6. Der Verjüngungsbereich ist konisch mit einem sehr geringen spitzen Winkel. Am vorderen Einführende 6 ist der insgesamt einstückig ausgebildete Führungsdraht mit einer Erweiterung 7 versehen, die im dargestellten Ausführungsbeispiel Tropfenform hat und am freien Ende teils kugelförmig ausgebildet ist, auf ihrer rückwärtigen Seite wie das Ende eines Tropfens kontinuierlich in den konischen Bereich 4 übergeht.

Der Maximaldurchmesser des Führungsdrahtes liegt bei einer bevorzugten Ausführungsform etwas unter oder bei 1 mm, der Minimaldurchmesser bei 0,2 mm. Die Länge des Übergangsbereiches liegt bei 70 mm, kann aber beispielsweise auch bis zu 250 mm betragen.

Durch die konische Ausgestaltung erhält der Führungsdraht 4 in diesem Übergangsbereich eine höhere Biegeflexibilität als in seinem Bereich 2 konstanten Querschnitts, so daß insbesondere der konische Übergangsbereich 4 allen Biegungen und Verästelungen in Gefäßen und Organen folgen kann. Durch die Abrundung der vorderen Erweiterung werden Verletzungen ausgeschlossen. Ein Blockieren einer mittels eines solchen Führungsdrahtes eingeführten Kanüle ist praktisch ausgeschlossen.

Bei der Ausgestaltung der Figur 2 sind der rückwärtige Hauptbereich 2 und das Einführende 6 mit der Erweiterung 7 wie bei der Ausgestaltung der Figur 1 ausgebildet. Eine erhöte Flexibilität im vorderen Bereich 4a zwischen dem Hauptabschnitt 2 und dem Einführende 6 wird hier durch periodisch wechselnde Erweiterungsbereiche 8 und Verengungen 9 erreicht. Die Länge einer Periode beträgt in einem bevorzugten Ausführungsbeispiel 5 mm, kann aber auch länger sein.

Während vorstehend Führungsdrähte mit kreisförmigem Querschnitt erläutert wurden, kann der Querschnitt auch andere Formen, wie Oval- oder Ellipsenorm, haben.

Durch die einstückige Ausbildung des erfindungsgemäßen Führungsdrahtes weist dieser eine äußerst hohe Zugfestigkeitauf, so daß verhindert wird, daß Teile, insbesondere die Erweiterung 7, sich lösen können. Bei einem Versuch wurde beispielsweise festgesteltl, daß bei einer die Erweiterung 7 in eine Stahlkanüle einziehenden Zugkraftausübung mit einer Öffnung der Kanüle geringfügig unter den Querschnittsabmessungen der Erweiterung 7 bei einem Minimaldurchmesser des Führungsdrahtes an seiner geringsten Stelle von 0,25 mm nicht etwa die Erweiterung 7 abriß, sondern diese die Öffnung der Stahlkanüle erweiterte und damit der Führungsdraht durch diese hindurchgezogen werden konnte.

Während verschiedene hochflexible Materialien mit hoher Zugfestigkeit verwendet werden können, werden in bevorzugter Ausgestaltung Nickel-Titan-Legierungen eingesetzt, insbesondere solche, die unter der Bezeichnung Nitinol bekannt sind, da sie neben der hohen Zugfestigkeit auch eine hohe elastische Biegefähigkeit aufweisen und praktisch auch bei wesentlich größeren Kräften, als sie unter normalen Umständen auftreten nicht irreversibel, plastisch geknickt werden können. Grundsätzlich kommen aber auch Kunststoffmaterialien, insbesondere faserverstärkte, wie mittels Graphitfasern verstärkte Materialien in Frage.

Wesentlich ist auch, daß die Außenkontur des vollständig einstückigen erfindungsgemäßen Führungsdrahtes glatt ist und im Umfangsbereich keine spitzen Winkel in der Außenkontur vorhanden sind, wie dies bei gewendelten Führungsdrähten der Fall ist, wo zwischen zwei nebeneinander liegenden Windungen die radiale Öffnung zwischen diesen einen gegen 0 gehenden Winkel aufweist, so daß sich hier Gegenstände, wie beispielsweise die Spitzen von schräg angeschärften Kanülen, verhaken können, was dort zu einem Abreißen führen kann.

Während die erfindungsgemäßen Führungsdrähte beispielsweise auch durch Schleifen und Polieren in der dargestellten Weise hergestellt werden können, erfolgt die Herstellung in bevorzugter Ausgestaltung bei aus Metall, wie Nickel-Titan-Legierungen, bestehenden Führungsdrähten durch Ätzen in einem Ätzbad, wie Kieselsäure. Die Führungsdrähte werden dabei über einen Längenbereich, der ihrer Querschnittserweiterung von der engsten Stelle bis zu der Stelle mit dem größten Stelle entspricht, kontinuierlich aus einem Ätzbad herausgezogen, wodurch sich die kontinuierliche Verjüngung ergibt. Andere Bereich können dabei durch gegenüber dem Ätzbar resistente Beschichtungen, wie einen Fotolack, abgedeckt und geschützt sein; gegebenenfalls kann auch das Durchziehen durch Dichtungen erfolgen. Auch wenn die Verjüngungen durch Ätzen erzielt werden, kann eine Endbehandlung mittels Polieren erfolgen.

So wird beispielsweise der Führungsdraht der Figur 1 derart hergestellt, daß zunächst der Verjüngungsbereich 4 durch eine resistente Schicht abgedeckt wird und der rückwärtige Tropfenbereich 11 erzeugt wird, indem das vordere, freie Ende des herzustellenden Führungsdrahtes mit der Erweiterung 7 langsam kontinuierlich aus einem Ätzbad herausgeschoben wird, wobei der (noch nicht verjüngte) Übergangsbereich 4 durch eine Dichtung im Boden des Ätzbades geschoben wird.

Anschließend wird das Einführende 6 mit der Erweiterung 7 bis zur durch den vorstehend beschriebenen Vorgang jüngsten Stelle mit der Schutzschicht versehen, während die Schutzschicht über dem Verjüngungsbereich 4 entfernt wird. Der Führungsdraht wird über die gewünschte Länge des Verjüngungsbereiches 4 in das Ätzbad eingebracht und dann langsam kontinuierlich zum rückwärtigen Ende 3 hin aus diesem herausgezogen. Hierdurch bleiben Bereiche des Verjüngungsabschnitts 4 nahe dem freien Einführende 6 länger im Ätzbad als Bereiche nahe dem Hauptabschnitt 2, so daß die erstgenannten Bereiche stärker dem Ätzbad ausgesetzt sind und damit eine größere Querschnittsreduzierung erfahren als die letztgenannten Bereiche.

Entsprechend kann abschnittsweise zur Herstellung der Verjüngungen und Erweiterungen 8, 9 des Führungsdrahtes der Figur 2 vorgegangen werden.

## Patentansprüche

1. Führungsteil zur Katheterisierung, das massiv und einstückig aus elastischem Material besteht, mit einem rückwärtigen Hauptabschnitt (2) konstanten Querschnitts und mit einem vorderen Einführende (6) mit abgerundeter Stirnseite, das einen größeren Durchmesser aufweist als der minimale Durchmesser eines Übergangsbereichs (4), der sich von einer Stelle minimalen Querschnitts unmittelbar hinter dem Einführende (6) von diesem fort über seine gesamte Länge sich kontinuierlich erweiternd verläuft, dadurch gekennzeichnet, daß das Einführende (6) die Form eines Tropfens aufweist, der an seinem rückwärtigen Ende stetig in den Übergangsbereich (4) übergeht.

2. Führungsteil zur Katheterisierung, das massiv und einstückig aus elastischem Metall besteht, mit einem rückwärtigen Hauptabschnitt (2) konstanten Querschnitts und mit einem vorderen Einführende (6) mit abgerundeter Stirnseite, das einen größeren Durchmesser aufweist als der minimale Durchmesser eines Übergangsbereichs (4), dadurch gekennzeichnet, daß das Einführende (6) die Form eines Tropfens aufweist, der an seinem rückwärtigen Ende stetig in den Übergangsbereich (4) übergeht, und daß des Führungsteil mehrere sich kontinuierlich periodisch erweiternde und verjüngende Abschnitte (8,9) aufweist.

3. Führungsteil nach Anspruch 1 oder 2, dadurch gekennzeichnet, daß der Minimaldurchmesser weniger als ein Viertel des Maximaldurchmessers beträgt.

4. Führungsteil nach Anspruch 1, dadurch gekennzeichnet, daß der Übergangsbereich (4) konisch ausgebildet ist.

5. Führungsteil nach Anspruch 5, dadurch gekennzeichnet, daß der sich von der Stelle geringsten Querschnitts hinter dem Einführende (6) von diesem forterstreckende Übergangsbereich (4) mindestens zehnmal so lang ist wie der Abschnitt von der Stelle geringsten Querschnitts unmittelbar hinter dem Einführende bis zur Stirnseite desselben.

6. Führungsteil nach einem der vorangehenden Ansprüche, dadurch gekennzeichnet, daß der sich von der Stelle geringsten Querschnitts hinter dem Einführende (6) von diesem forterstreckende Übergangsbereich (4) mindestens zwanzigmal so lang ist wie der Abschnitt von der Stelle geringsten Querschnitts unmittelbar hinter dem Einführende bis zur Stirnseite desselben.

7. Führungsteil nach einem der Ansprüche 2 bis 3, dadurch gekennzeichnet, daß der Übergangsbereich (4a) mehrere periodisch sich erweiternde und verjüngende Abschnitte (8,9) aufweist.

8. Führungsteil nach Anspruch 7, dadurch gekennzeichnet, daß der Übergangsbereich (4a) mindestens drei sich periodisch erweiternde und verjüngende Abschnitte (8,9) aufweist.

9. Führungsteil nach Anspruch 7 oder 8, dadurch gekennzeichnet, daß die Periode der sich erweiternden und verjüngenden Abschnitte (8,9) eine Länge zwischen 3 und 9 mm hat.

10. Führungsteil nach einem der vorangehenden Ansprüche, gekennzeichnet durch aus Massivmaterial abgetragene Abschnitte im Übergangsbereich (4) zwischen Einführende (6) und Hauptabschnitt (2).

11. Führungsteil nach Anspruch 10, dadurch gekennzeichnet, daß es abgeschliffene Abschnitte aufweist.

12. Führungsteil nach Anspruch 10, dadurch gekennzeichnet, daß es abgeätzte Abschnitte aufweist.

13. Führungsteil nach einem der Ansprüche 1 bis 13, dadurch gekennzeichnet, daß es aus einer Nickel-Titan-Legierung besteht.

14. Führungsteil nach einem der vorangehenden Ansprüche, dadurch gekennzeichnet, daß es zumindestens in seinem vorderen Bereich (6,4) in Abhängigkeit von der Temperatur oberhalb bzw. unterhalb eines vorgegebenen Temperaturbereichs eine unterschiedliche Konfiguration einnimmt (Memory-Eigenschaft).

15. Führungsteil nach einem der vorangehenden Ansprüche, dadurch gekennzeichnet, daß die vordere Stirnseite des Einführendes teilkugelförmig, insbesondere halbkugelförmig ausgebildet ist.

16. Führungsteil nach einem der vorangehenden Ansprüche, dadurch gekennzeichnet, daß das vordere Einführende in Axialerstreckung ausschließlich Bereiche mit sich ändernden Querschnittsabmessungen aufweist.

17. Verfahren zum Herstellen eines Führungsteils, nach einem der vorangehenden Ansprüche, dadurch gekennzeichnet, daß ein stabförmiges, einstückiges Teil mit glatter Außenwandung aus flexiblem Metall in Teilbereichen zur Erzeugung von Querschnittsverminderungen abgeschliffen wird.

18. Verfahren nach Anspruch 17, dadurch gekennzeichnet, daß die Querschnittsverminderungen mit kontinuierlichen Übergangsbereichen hergestellt werden.

19. Verfahren zum Herstellen eines Führungsteils, nach einem der Ansprüche 1 bis 16, dadurch gekennzeichnet, daß ein einstückiges, stabförmiges Teil mit glatter Außenwandung aus flexiblem Metall zumindestens einmal wenigstens über einen Teil seiner Länge kontinuierlich aus einem sein Material angreifenden Ätzbad in eine nichtätzende Umgebung gezogen wird.

20. Verfahren nach Anspruch 19, dadurch gekennzeichnet, daß vor dem Schritt des Anspruchs 19 zumindestens Teilbereiche des stabförmigen Teils mit einer gegen das Ätzbad resistenten Beschichtung versehen wird.

21. Verfahren nach Anspruch 18 oder 19, dadurch gekennzeichnet, daß das stabförmige Teil mit konstanter Geschwindigkeit aus dem Ätzbad herausgezogen wird.

22. Verfahren nach einem der Ansprüche 19 bis 21, dadurch gekennzeichne,t daß das stabförmige Teil zumindestens durch eine das Ätzbad gegen eine nichtätzende Umgebung abdichtende Dichtung hindurchgezogen wird.

23. Verfahren nach einem der Ansprüche 19 bis 22, dadurch gekennzeichnet, daß ein massives stabförmiges Teil aus dem Ätzbad herausgezogen wird.

24. Verfahren nach einem der Ansprüche 19 bis 23, dadurch gekennzeichnet, daß ein rohrförmiges Teil aus dem Ätzbad herausgezogen wird.

25. Verfahren nach einem der Ansprüche 19 bis 24, dadurch gekennzeichnet, daß die Temperatur des Ätzbades konstant gehalten wird.

## Claims

1. Guide part for catheterization, which is solid and is made in one piece from elastic material, having a rear main portion (2) of constant cross-section and with a front insertion end (6) with a rounded front side, which has a larger diameter than the minimum diameter of a transition area (4), which from a minimum cross-section point immediately behind the insertion end (6) passes in continuously widening manner therefrom over its entire length, characterized in that the insertion end (6) is in the shape of a drop, which at its rear end passes continuously into the transition area (4).

2. Guide part for catheterization, which is solid and is made in one piece from elastic material, having a rear main portion (2) of constant cross-section and with a front insertion end (6) with a rounded front side, which has a larger diameter than the minimum diameter of a transition area (4), characterized in that the insertion end (6) is shaped like a drop, which at its rear end passes continuously into the transition area (4) and that the guide part has several continuously periodically widening and narrowing portions (8, 9).

3. Guide part according to claim 1 or 2, characterized in that the minimum diameter is less than a quarter of the maximum diameter.

4. Guide part according to claim 1, characterized in that the transition area (4) has a conical construction.

5. Guide part according to claim 4 , characterized in that the transition area (4) extending from the point with the smallest cross-section behind the insertion end (6) and away from the latter is at least ten times as long as the portion from the point of minimum cross-section immediately behind the insertion end up to the front side thereof.

6. Guide part according to one of the preceding claims, characterized in that the transition area (4) extending from the point of minimum cross-section behind the insertion end (6) and away therefrom is at least twenty times as long as the portion from the point of minimum cross-section immediately behind the insertion end up to the front side thereof.

7. Guide part according to one of the claims 2 and 3, characterized in that the transition area (4a) has several periodically widening and narrowing portions (8, 9).

8. Guide part according to claim 7, characterized in that the transition area (4a) has at least three periodically widening and narrowing portions (8, 9).

9. Guide part according to claim 7 or 8, characterized in that the period of the widening and narrowing portions (8, 9) has a length between 3 and 9 mm.

10. Guide part according to one of the preceding claims, characterized by portions removed from solid material in the transition area (4) between the insertion end (6) and main portion (2).

11. Guide part according to claim 10, characterized in that it has abraded portions.

12. Guide part according to claim 10, characterized in that it has etched portions.

13. Guide part according to one of the claims 1 to 13, characterized in that it comprises a nickel-titanium alloy.

14. Guide part according to one of the preceding claims, characterized in that at least in its front area (6, 4), as a function of the temperature above or below a predetermined temperature range, it assumes a different configuration (memory characteristic).

15. Guide part according to one of the preceding claims, characterized in that the front side of the insertion end has a part spherical, particularly hemispherical construction.

16. Guide part according to one of the preceding claims, characterized in that the front insertion end in axial extension exclusively has areas with varying cross-sectional dimensions.

17. Method for the manufacture of a guide part according to one of the preceding claims, characterized in that a rod-shaped, one-piece part with a smooth outer wall of flexible material is abraded in partial areas for producing cross-sectional reductions.

18. Method according to claim 17, characterized in that the cross-sectional reductions are produced with continuous transition areas.

19. Method for the manufacture of a guide part according to one of the claims 1 to 16, characterized in that a one-piece, rod-shaped part with a smooth outer wall of flexible material is drawn at least once at least over part of its length continuously out of an etching bath attacking its material into a non-etching environment.

20. Method according to claim 19, characterized in that prior to the step according to claim 19, at least partial areas of the rod-shaped part are provided with a coating resistant to the etching bath.

21. Method according to claim 18 or 19, characterized in that the rod-shaped part is drawn out of the etching bath at a constant speed.

22. Method according to one of the claims 19 to 21, characterized in that the rod-shaped part is drawn at least once through a seal sealing the etching bath against a non-etching environment.

23. Method according to one of the claims 19 to 22, characterized in that a solid, rod-shaped part is drawn out of the etching bath.

24. Method according to one of the claims 19 to 23, characterized in that a tubular part is drawn out of the etching bath.

25. Method according to one of the claims 19 to 24, characterized in that the etching bath temperature is kept constant.

## Revendications

1. Pièce de guidage pour la cathétérisation réalisée massive et d'une seule pièce dans un matériau élastique, comportant une partie principale (2) arrière de section constante et une extrémité avant d'introduction (6) à face frontale arrondie, dont le diamètre est supérieur au diamètre minimal d'une zone de transition (4) qui s'étend à partir d'un endroit à section minimale existant immédiatement derrière l'extrémité d'introduction (6) en s'élargissant de façon continuelle sur toute sa longueur, caractérisée en ce que l'extrémité d'introduction (6) affecte la forme d'une goutte qui par son extrémité arrière se confond progressivement avec la zone de transition (4).

2. Pièce de guidage pour la cathétérisation, réalisée massivement et d'une seule pièce dans un métal élastique, comportant une partie principale (2) arrière de section constante et une extrémité avant d'introduction (6) à face frontale arrondie, dont le diamètre est supérieur au diamètre minimal d'une zone de transition (4), caractérisée en ce que l'extrémité d'introduction (6) affecte la forme d'une goutte qui par son extrémité arrière se confond progressivement avec la zone de transition (4), et en ce que la pièce de guidage présente plusieurs parties (8,9) s'élargissant et se rétrécissant de façon continue de manière périodique.

3. Pièce de guidage selon la revendication 1 ou 2, caractérisée en ce que le diamètre minimal représente moins d'un quart du diamètre maximal.

4. Pièce de guidage selon la revendication 1, caractérisée en ce que la zone de transition (4) est de forme conique.

5. Pièce de guidage selon la revendication 4, caractérisée en ce que la zone de transition (4) s'étendant à partir de l'endroit à section minimale situé derrière l'extrémité d'introduction (6) présente une longueur égale à au moins dix fois la longueur de la partie comprise entre l'endroit à section minimale situé immédiatement derrière l'extrémité d'introduction jusqu'à la face frontale de celle-ci.

6. Pièce de guidage selon l'une quelconque des revendications précédentes, caractérisée en ce que la zone de transition (4) s'étendant à partir de l'endroit à section minimale derrière l'extrémité d'introduction (6) présente une longueur égale à au moins vingt fois la longueur de la partie comprise entre l'endroit à section minimale immédiatement derrière l'extrémité d'introduction jusqu'à la face frontale de celle-ci.

7. Pièce de guidage selon l'une quelconque des revendications 2 à 3, caractérisée en ce que la zone de transition (4a) présente plusieurs parties (8,9) s'élargissant et se rétrécissant périodiquement.

8. Pièce de guidage selon la revendication 7, caractérisée en ce que la zone de transition (4a) présente au moins trois parties (8,9) s'élargissant et se rétrécissant périodiquement.

9. Pièce de guidage selon la revendication 7 ou 8, caractérisée en ce que la longueur de la période des parties (8,9) s'élargissant et se rétrécissant se situe entre 3 et 9 mm.

10. Pièce de guidage selon l'une quelconque des revendications précédentes, caractérisée par des parties en matériau massif abrasé dans la zone de transition (4) entre l'extrémité d'introduction (6) et la partie principale (2).

11. Pièce de guidage selon la revendication 10, caractérisée en ce qu'elle présente des parties meulées.

12. Pièce de guidage selon la revendication 10, caractérisée en ce qu'elle présente des parties corrodées.

13. Pièce de guidage selon l'une quelconque des revendications 1 à 13, caractérisée en ce qu'elle est composée d'un alliage de nickel et de titane.

14. Pièce de guidage selon l'une quelconque des revendications précédentes, caractérisée en ce que, en fonction d'une température située au-dessus ou en-dessous d'une plage de température prédéfinie, elle affecte une forme différente (propriété de mémoire), au moins dans sa zone avant (6,4).

15. Pièce de guidage selon l'une quelconque des revendications précédentes, caractérisée en ce que la face frontale avant de l'extrémité d'introduction présente une forme partiellement sphérique, en particulier hémisphérique.

16. Pièce de guidage selon l'une quelconque des revendications précédentes, caractérisée en ce que l'extrémité avant d'introduction présente dans la direction axiale uniquement des zones dont les dimensions de section sont variables.

17. Procédé de fabrication d'une pièce de guidage selon l'une quelconque des revendications précédentes, caractérisé en ce qu'un élément d'une seule pièce en forme de tige à surface extérieure lisse en métal flexible est abrasé dans des zones partielles afin de réaliser des réductions de section.

18. Procédé selon la revendication 17, caractérisé en ce que les réductions de section sont réalisées avec des zones de transition continues.

19. Procédé de fabrication d'une pièce de guidage selon l'une quelconque des revendications 1 à 16, caractérisé en ce qu'un élément d'une seule pièce en forme de tige à surface extérieure lisse en métal flexible est retiré au moins une fois au moins sur une partie de sa longueur en continu d'un bain caustique attaquant le matériau dont il est composé et placé dans un environnement non caustique.

20. Procédé selon la revendication 19, caractérisé en ce qu'avant l'étape décrite dans la revendication 19, des portions partielles au moins de la pièce en forme de tige sont recouvertes d'une couche résistante au bain caustique.

21. Procédé selon la revendication 18 ou 19, caractérisé en ce que la pièce en forme de tige est retirée à vitesse constante du bain caustique.

22. Procédé selon l'une quelconque des revendications 19 à 21, caractérisé en ce que la pièce en forme de barre est passée au travers d'au moins une séparation d'étanchéité séparant de manière étanche le bain caustique d'un environnement non caustique.

23. Procédé selon l'une quelconque des revendications 19 à 22, caractérisé en ce que l'on retire une pièce massive en forme de tige du bain caustique.

24. Procédé selon l'une quelconque des revendications 19 à 23, caractérisé en ce que l'on retire une pièce tubulaire du bain caustique.

25. Procédé selon l'une quelconque des revendications 19 à 24, caractérisé en ce que la température du bain caustique est maintenue constante.
